# EUROPÄISCHE PATENTSCHRIFT

(11) **EP 0 672 073 B1**
(45) Veröffentlichungstag und Bekanntmachung des Hinweises auf die Patenterteilung: **03.07.1996**
(21) Anmeldenummer: 94901947.5
(22) Anmeldetag: 02.12.1993
(51) Int. Cl.: C08F 218/04, D04H 1/64

(54) **VERFAHREN ZUR HERSTELLUNG EINES SIEGELBAREN, SELBSTVERNETZENDEN BINDEMITTELS**
PROCESS FOR PRODUCING A SEALABLE, SELF-CROSSLINKING BINDER
PROCEDE DE PRODUCTION D'UN LIANT AUTORETICULANT SCELLABLE

(30) Priorität: 03.12.1992 DE 4240731
(43) Veröffentlichungstag der Anmeldung: 20.09.1995
(73) Patentinhaber: Wacker-Chemie GmbH, D-81737 München (DE)
(72) Erfinder: LUMPP, Andreas, D-84489 Burghausen (DE); KÖGLER, Gerhard, D-84508 Burgkirchen/Hirten (DE); KÖNECKE, Anja, D-31303 Burgdorf (DE)
(74) Vertreter: Schuderer, Michael
(86) Internationale Anmeldenummer: EP9303386
(87) Internationale Veröffentlichungsnummer: WO9412549

(56) Entgegenhaltungen:
- US-A- 3 806 402
- US-A- 5 109 063

## Beschreibung

Die Erfindung betrifft ein Verfahren zur Herstellung eines siegelbaren, selbstvernetzenden Bindemittels, gemäß dem Verfahren hergestellte wäßrige Bindemitteldispersionen sowie deren Verwendung zur Imprägnierung und Beschichtung von Faservliesen.

Die Ausrüstung und Verfestigung von Fasermaterialien, wie Geweben, Vliesen und Watten aus Textilfasern und Garnen, durch Imprägnieren mit selbstvernetzenden, wäßrigen Kunststoffdispersionen ist bekannt. In der Regel enthalten diese Dispersionen als vernetzungsfähige Gruppen einpolymerisierte Einheiten von N-Methylolamiden der Acryl- und/oder Methacrylsäure und/oder deren Ether. Durch Trocknung der bindemittelhaltigen Fasermaterialien bei Temperaturen von 100 bis 160°C wird die wäßrige Phase verdampft und das Bindemittel vernetzt. Auf diese Weise gebundene Faservliese weisen hohe Festigkeiten auf, sowohl im trockenen als auch im nassen Zustand.

In der DE-AS 1594951 (US-A 3380851) wird zur Bindung von Fasermaterialien ein Terpolymer aus Vinylacetat, Ethylen und N-Methylolacrylamid beschrieben. Die Herstellung erfolgt nach dem Emulsionspolymerisationsverfahren, wobei Vinylacetat in der wäßrigen Emulsion vorgelegt, Ethylen aufgedrückt wird und nach Initiierung der Polymerisation das N-Methylolacrylamid zudosiert wird. Die DE-OS 2325677 (US-A 3843580) beschreibt selbstvernetzende Bindemittel für Faservliese auf der Basis wäßriger Dispersionen von Mischpolymerisaten, enthaltend Vinylchlorid, Ethylen und N-Methylolacrylamid. Die Herstellung erfolgt durch Aufdrücken von Vinylchlorid und Ethylen auf die initiatorhaltige Emulsion und Zudosierung von N-Methylolacrylamid während der Polymerisation. Mit den ebengenannten Bindemitteln sind Fasermaterialien mit hoher Naßfestigkeit zugänglich. Nachteilig ist allerdings, daß damit gebundene Faservliese, aufgrund des N-Methylolamid-Anteils nicht siegelfähig sind.

In vielen Anwendungsbereichen, beispielsweise bei der Herstellung von Papierwindeln oder Teppichen, wird nun die Anforderung gestellt, daß derartige verfestigte Fasermaterialien heißsiegelbar sind, das heißt bei erhöhter Temperatur und unter Druck sich das Fasermaterial über den Bindemittelanteil mit anderen Materialien, wie Papier, Textilien oder Kunststoff, verbinden läßt. Mit den obengenannten Bindemitteln auf der Basis von N-Methylol-haltigen Mischpolymerisaten ist dies nicht möglich.

In der EP-A 133277 (US-A 4481250) wird empfohlen selbstvernetzende, siegelbare Bindemittel einzusetzen, welche aus einem Gemisch aus Ethylen/Vinylacetat-Copolymer mit Ethylen/Vinylacetat/N-Methylolacrylamid-Copolymer bestehen. Nachteilig ist hierbei das relativ aufwendige Herstellungsverfahren im Vergleich mit Bindemitteldispersionen, welche nur eine Copolymerkomponente enthalten.

Die DE-A 3734752 (US-A 4912147) beschreibt selbstvernetzende, siegelbare Bindemitteldispersionen auf der Basis eines (Meth)acrylat-Copolymers, welches (Meth)acrylamidoglykolsäure-Einheiten enthält. Die Herstellung erfolgt in einem zweistufigen Verfahren, wobei der Anteil an (Meth)acrylamidoglykolsäure vorzugsweise im zweiten Schritt copolymerisiert wird. Copolymere, welche N-Methylolgruppen enthalten, werden als nicht siegelfähig beschrieben.

Der Erfindung lag die Aufgabe zugrunde, eine wäßrige Copolymerdispersion zur Verfügung zu stellen, mit der Fasermaterialien mit hoher Trockenfestigkeit und Naßfestigkeit gebunden werden und die Siegelbarkeit der gebundenen Fasermaterialien gewährleistet ist.

Gegenstand der Erfindung ist ein Verfahren zur Herstellung eines siegelbaren, selbstvernetzenden Bindemittels auf der Basis einer wäßrigen Dispersion von Polymerisaten enthaltend eines oder mehrere Monomere aus der Gruppe der Vinylester von unverzweigten oder verzweigten Alkylcarbonsäuren mit 1 bis 15 C-Atomen, welche 0.1 bis 5.0 Gew%, bezogen auf das Polymerisat, ethylenisch ungesättigte, vernetzend wirkende Comonomere aus der Gruppe der N-Alkylolamide und N-Alkoxyalkylamide ethylenisch ungesättigter Carbonsäuren und N-Methylolallylcarbamat enthalten, durch radikalische Polymerisation nach dem Emulsionspolymerisationsverfahren, dadurch gekennzeichnet, daß die Comonomeren des zu polymerisierenden Comonomergemisches, mit Ausnahme der vernetzend wirkenden Comonomerkomponente, teilweise vorgelegt und der Rest der vernetzerfreien Comonomermischung nach Initiierung der Polymerisation und Auspolymerisation der Vorlage zudosiert wird, und die Zugabe der ethylenisch ungesättigten, vernetzend wirkenden Comonomere erst ab dem Zeitpunkt erfolgt, zu dem mindestens 50 Gew% der nicht-vernetzenden Comonomere zum Polymerisationsansatz zudosiert worden sind.

Bevorzugte Vinylester sind Vinylacetat, Vinylpropionat, Vinylbutyrat, Vinyl-2-ethylhexanoat, Vinyllaurat, 1-Methylvinylacetat, Vinylpivalat und Vinylester von α-verzweigten Monocarbonsäuren mit 9 bis 10 C-Atomen, beispielsweise VeoVa9^{R} oder VeoVa10^{R}.

Die genannten Vinylester können gegebenenfalls mit α-Olefinen wie Ethylen oder Propylen, Vinylaromaten wie Styrol und/ oder mit 0.1 bis 20 Gew%, bezogen auf das Gesamtgewicht der Comonomerphase, Acrylsäureester bzw. Methacrylsäureester von Alkoholen mit 1 bis 10 C-Atomen, wie Methylacrylat, Methylmethacrylat, Ethylacrylat, Ethylmethacrylat, Propylacrylat, Propylmethacrylat, n-Butylacrylat, n-Butylmethacrylat, 2-Ethylhexylacrylat und/oder ethylenisch ungesättigten Dicarbonsäureestern wie Diisopropylfumarat copolymerisiert werden. Die Auswahl aus den genannten Monomeren wird dabei so getroffen, daß bei der Herstellung der Bindemitteldispersion Copolymerisate mit einer Glasübergangstemperatur Tg von -60°C bis +60°C erhalten werden.

Zur Copolymerisation geeignete ethylenisch ungesättigte, vernetzend wirkende Comonomere sind N-Alkylolamide ethylenisch ungesättigter Carbonsäuren wie N-Methylolacrylamid oder N-Methylolmethacrylamid, N-Alkoxyalkylamide ethylenisch ungesättigter Carbonsäuren wie N-(isobutoxymethyl)-acrylamid. Vorzugsweise wird N-Methylolacrylamid oder N-Methylolmethacrylamid zur Polymerisation eingesetzt. Die verwendeten Gewichtsmengen für die vernetzend wirkenden Comonomere betragen vorzugsweise von 0.1 bis 2.0 Gew%, bezogen auf das Gesamtgewicht der Comonomerphase.

Für die Polymerisation bevorzugte Comonomergemische sind solche, welche 75 bis 95 Gew% Vinylester, insbesonders Vinylacetat, sowie 5 bis 25 Gew% α-Olefin, insbesonders Ethylen, und 0.1 bis 2.0 Gew% vernetzend wirkendes Comonomer, insbesonders N-Methylolacrylamid, enthalten oder Comonomergemische enthaltend 50 bis 70 Gew% Vinylester, insbesonders Vinylacetat, 10 bis 30 Gew% Vinylester einer α-verzweigten Carbonsäure, insbesonders VeoVa9^{R} und/oder VeoVa10^{R}, 5 bis 25 Gew% Ethylen und 0.1 bis 2.0 Gew% vernetzend wirkendes Comonomer, insbesonders N-Methylolacrylamid. Vorteilhafterweise enthalten die genannten Vinylester/Ethylen-Comonomergemische noch 0.1 bis 20 Gew% Acrylsäureester, beispielsweise Butylacrylat, oder Diisopropylfumarat. Die Prozentangaben sind dabei jeweils bezogen auf das Gesamtgewicht des Comonomergemisches und ergänzen sich jeweils auf 100 Gewichtsprozent.

In einer besonders bevorzugten Ausführungsform enthalten die Comonomergemische noch 0.05 bis 3.0 Gew%, bezogen auf das Gesamtgewicht des Comonomergemisches, Hilfsmonomere aus der Gruppe der ethylenisch ungesättigten Carbonsäuren, vorzugsweise Acrylsäure oder Methacrylsäure, aus der Gruppe der ethylenisch ungesättigten Carbonsäureamide, vorzugsweise Acrylamid, aus der Gruppe der ethylenisch ungesättigten Sulfonsäuren bzw. deren Salze, vorzugsweise Vinylsulfonsäure, und/oder aus der Gruppe der mehrfach ethylenisch ungesättigten Comonomeren, beispielsweise Divinyladipat, Diallylmaleat, Allylmethacrylat oder Triallylcyanurat.

Bei der erfindungsgemäßen Verfahrensweise zur Herstellung der Bindemitteldispersion in radikalischer Emulsionspolymerisation werden die Comonomeren des zu polymerisierenden Comonomergemisches, mit Ausnahme der vernetzend wirkenden Comonomerkomponente, teilweise vorgelegt und der Rest zudosiert. Vorzugsweise werden 5 bis 25 Gew% des eben definierten Comonomergemisches vorgelegt und der Rest der Mischung nach Initiierung der Polymerisation zudosiert. Die Zugabe der vernetzend wirkenden Comonomerkomponente erfolgt vorzugsweise ab dem Zeitpunkt, zu dem 50 bis 99.5 Gew%, besonders bevorzugt ab dem Zeitpunkt zu dem 65 bis 95 Gew% der nicht-vernetzenden Comonomere zudosiert werden sind.

Die radikalische Emulsionspolymerisation kann in den üblichen Polymerisationsreaktoren, bei der Copolymerisation mit gasförmigen Monomeren wie Ethylen, in den üblichen Druckreaktoren durchgeführt werden. Die Reaktionstemperatur beträgt üblicherweise von 0 bis 80°C, vorzugsweise von 40 bis 80°C. Bei der Copolymerisation mit Ethylen kann der zu Beginn der Polymerisation eingestellte Ethylendruck über die gesamte Reaktionsdauer beispielsweise durch Nachdrücken von Ethylen konstant gehalten werden. Es ist aber auch möglich, die anfangs in den Reaktor eingebrachte Ethylenmenge nicht zu ergänzen oder den Ethylendruck während der Polymerisation innerhalb der genannten Grenzen zu variieren.

Die Polymerisation wird unter Initiierung mittels üblicher wasserlöslicher Radikalbildner durchgeführt, die vorzugsweise in Mengen von 0.05 bis 3.0 Gew%, bezogen auf das Gesamtgewicht der Comonomere, eingesetzt werden. Dies sind insbesonders Persulfate wie Ammoniumperoxodisulfat oder Kaliumpersulfat, Hydroperoxide wie t-Butylhydroperoxid und Perphosphate. Diese Verbindungen können entweder direkt durch Temperaturerhöhung oder bei niederen Temperaturen durch Einsatz von Reduktionsmitteln aktiviert werden. Bevorzugt wird hierbei die Aktivierung der Radikalbildner durch Reduktionsmittel wie Natriumformaldehydsulfoxylat, Natriumsulfit, Natriumhydrogensulfit, Dithionit oder Ascorbinsäure. Besonders bevorzugt ist die Verwendung von Ascorbinsäure als Reduktionsmittel. Vorzugsweise werden eine oder beide Redoxkatalysator-Komponenten während der Polymerisation dosiert.

Der bevorzugte pH-Bereich für die Polymerisationsreaktion ist 3 bis 6.5. Die pH-Einstellung oder Regelung des pH-Wertes erfolgt mit Säuren wie Schwefelsäure, Ameisensäure, Essigsäure und mit Basen wie Ammoniak, Natronlauge, Kalilauge, Aminen oder durch Verwendung von Puffersubstanzen wie Alkaliacetat, Alkalicarbonat, Alkalidiphosphaten.

Als Dispergiermittel können die üblicherweise bei der Emulsionspolymerisation verwendeten ionischen und nichtionischen Emulgatoren und Schutzkolloide eingesetzt werden. Vorzugsweise werden 1 bis 6 Gew%, bezogen auf das Gesamtgewicht der Monomeren, an Emulgator eingesetzt. Geeignet sind beispielsweise anionische Tenside, wie Alkylsulfate mit einer Kettenlänge von 8 bis 18 C-Atomen, Alkyl- und Alkylarylethersulfate mit 8 bis 18 C-Atomen im hydrophoben Rest und bis zu 40 Ethylen- oder Propylenoxideinheiten, Alkyl- oder Alkylarylsulfonate mit 8 bis 18 C-Atomen, Ölsäuresulfonate, Ester und Halbester der Sulfobernsteinsäure mit einwertigen Alkoholen oder Alkylphenolen. Geeignete nichtionische Tenside sind beispielsweise Alkylpolyglykolether oder Alkylarylpolyglykolether mit 8 bis 40 Ethylenoxideinheiten.

Gegebenenfalls können Schutzkolloide, vorzugsweise in Mengen bis zu 15 Gew%, bezogen auf das Gesamtgewicht der Monomeren, eingesetzt werden. Beispiele hierfür sind Vinylalkohol/Vinylacetat-Copolymere mit einem Gehalt von 80 bis 100 Mol% Vinylalkoholeinheiten, Polyvinylpyrrolidone mit einem Molekulargewicht von 5000 bis 400000, Hydroxyethylcellulosen mit einem Substitutionsgrad von 1.5 bis 3.

Die nach dem erfindungsgemäßen Verfahren hergestellten Polymerdispersionen weisen im allgemeinen einen Festgehalt von 35 bis 65 Gew% auf. Sie eignen sich als Bindemittel für gewebte oder nicht gewebte flächige textile Gebilde aus üblichen natürlichen oder synthetischen Fasern wie Wolle, Baumwolle, Viscose, Polyamiden, Polyestern, Polypropylen oder Polyethylen, denen sie Festigkeit im trockenen und nassen Zustand bei gleichzeitiger Heißsiegelbarkeit verleihen.

Bei der Anwendung werden sie in üblicher Weise durch Imprägnieren, Schaumimprägnieren, Sprühen, Pflatschen, Streichen oder Bedrucken aufgetragen. Gegebenenfalls nach Abtrennen des überschüssigen Bindemittels durch beispielsweise Abquetschen, werden die gebundenen textilen Gebilde bei Temperaturen von 80 bis 200°C, vorzugsweise zwischen 120 und 180°C, getrocknet und dann getempert.

Zu dem erfindungsgemäßen Bindemittel können noch Pigmente, Antioxidantien, Farbstoffe, Weichmacher, Filmbildehilfsmittel, Füllstoffe, Flammschutzmittel, Schaumbildehilfsmittel, Schauminhibitoren, Netzmittel, Thermosensibilisierungsmittel, Antistatika, Biozide, Griffverbesserungsmittel, zusätzliche Vernetzer oder Katalysatoren zur eventuell notwendigen Beschleunigung der Vernetzungsreaktion in dazu üblichen Mengen zugegeben werden. Gute Gebrauchseigenschaften der gebundenen Substrate werden bei einem Binderauftrag von vorzugsweise 10 bis 60 Gew% erreicht.

Die mit den erfindungsgemäß hergestellten Polymerdispersionen behandelten Substrate wie Vliesstoffe zeichnen sich durch eine Kombination von guter Naßfestigkeit mit guter Heißsiegelbarkeit und einem sehr niedrigen Formaldehydgehalt aus. Aufgrund der Heißsiegelbarkeit vereinfacht sich die Weiterverarbeitung der mit den Polymerdispersionen behandelten Substrate. Diese können unter Anwendung von Druck und Wärme mit sich selbst oder anderen Substraten wie Textilien, Kunststoffen, Holz, Papier oder Metall aufgesiegelt werden, wobei in der Regel mindestens ein Arbeitsgang, beispielsweise Nähen, Kleberauftrag oder mechanische Befestigung eingespart wird.

Ein typisches Einsatzgebiet für die erfindungsgemäß hergestellten Polymerdispersionen ist die Fertigung von Hüllvliesen von Windeln oder Binden, bei denen das Vlies auch im nassen Zustand eine gewisse mechanische Festigkeit aufweisen soll und andererseits mittels Wärme und Druck an eine äußere Schutzfolie aufgesiegelt werden soll.

Die nachfolgenden Beispiele dienen zur weiteren Erläuterung der Erfindung.

### Beispiel 1:

In einem Druckreaktor wurden 3.0 l Wasser, 44.7 g einer 10 Gew%-igen wäßrigen Ameisensäurelösung, 3.80 kg einer 3.3 Gew%-igen wäßrigen Dispersion einer Hydroxyethylcellulose (Natrosol 250 GR), 718 g einer 25 Gew%-igen Lösung eines Isotridecylalkohol-ethylenoxids (Genapol X360) und 20.3 g einer 25 Gew%-igen wäßrigen Vinylsulfonat-Lösung vorgelegt. In die Vorlage wurden 40.2 g Butylacrylat, 797 g VeoVa10 (Vinylester von α-verzweigten Monocarbonsäuren mit 10 C-Atomen) und 538 g Vinylacetat einemulgiert. Es wurde auf 50°C aufgeheizt und Ethylen mit einem Druck von 60 bar aufgedrückt. Nach Erreichen des Temperaturgleichgewichtes wurden eine Lösung von 13.5 g Ammoniumperoxodisulfat in 546 g Wasser mit 76.2 g/h und eine Lösung von 6.78 g Ascorbinsäure in 543 g Wasser mit 74.8 g/h zudosiert. Nach Auspolymerisation der Vorlage wurde über einen Zeitraum von 5 Stunden ein Gemisch aus 5.44 kg Vinylacetat, 805 g VeoVa10 und 121 g Butylacrylat zudosiert. Nach Beendigung der Dosierung des Monomergemisches wurden 160 g N-Methylolacrylamid in 307 g Wasser über einen Zeitraum von 30 Minuten zudosiert.
Nach Beendigung der Polymerisation resultierte eine wäßrige Dispersion mit einem Festgehalt von 52 Gew% und einer Copolymerzusammensetzung aus 11.1 Gew% Ethylen, 67.8 Gew% Vinylacetat, 18.2 Gew% VeoVa10, 1.8 Gew% Butylacrylat, 0.1 Gew% Vinylsulfonat und 1.0 Gew% N-Methylolacrylamid.

### Vergleichsbeispiel 1:

In einem Druckreaktor wurden 2.7 l Wasser, 44.4 g einer 10 Gew%-igen wäßrigen Ameisensäurelösung, 3.77 kg einer 3.3 Gew%-igen wäßrigen Dispersion einer Hydroxyethylcellulose (Natrosol 250 GR), 606 g einer 29.4 Gew%-igen Lösung eines Isotridecyl-Ethylenoxidsulfats (Rewopol V3105), 20.2 g einer 25 Gew%-igen wäßrigen Vinylsulfonat-Lösung und 32.0 g einer 48 Gew%-igen wäßrigen N-Methylolacrylamid-Lösung vorgelegt. In die Vorlage wurden 39.9 g Butylacrylat, 791 g VeoVa10 (Vinylester von α-verzweigten Monocarbonsäuren mit 10 C-Atomen) und 534 g Vinylacetat einemulgiert. Es wurde auf 50°C aufgeheizt und Ethylen mit einem Druck von 60 bar aufgedrückt. Nach Erreichen des Temperaturgleichgewichtes wurden eine Lösung von 13.4 g Ammoniumperoxodisulfat in 525 g Wasser mit 66 g/h und eine Lösung von 6.7 g Ascorbinsäure in 532 g Wasser mit 65 g/h zudosiert. Nach Auspolymerisieren der Vorlage wurden über einen Zeitraum von 5 Stunden ein Gemisch aus 5.4 kg Vinylacetat, 799 g VeoVa10 und 120 g Butylacrylat sowie 160 g N-Methylolacrylamid in 307 g Wasser zudosiert.
Nach Beendigung der Polymerisation resultierte eine wäßrige Dispersion mit einem Festgehalt von 52 Gew% und einer Copolymerzusammensetzung aus 11.1 Gew% Ethylen, 67.8 Gew% Vinylacetat, 18.2 Gew% VeoVa10, 1.8 Gew% Butylacrylat, 0.1 Gew% Vinylsulfonat und 1.0 Gew% N-Methylolacrylamid.

### Beispiel 2:

Es wurde analog Beispiel 1 vorgegangen, mit dem Unterschied, daß 320 g N-Methylolacrylamid zudosiert wurden und ein Copolymer mit einem N-Methylolacrylamid-Gehalt von 2.0 Gew% resultierte.

### Vergleichsbeispiel 2:

Es wurde analog Beispiel 1 vorgegangen, mit dem Unterschied, daß kein N-Methylolacrylamid zugegeben wurde.

### Beispiel 3:

In einem Druckreaktor wurden 2.5 l Wasser, 38.7 g einer 10 Gew%-igen wäßrigen Ameisensäurelösung, 3.29 kg einer 3.3 Gew%-igen wäßrigen Dispersion einer Hydroxyethylcellulose (Natrosol 250 GR), 622 g einer 25 Gew%-igen Lösung eines Isotridecylalkohol-ethylenoxids (Genapol X360) und 17.6 g einer 25 Gew%-igen wäßrigen Vinylsulfonat-Lösung vorgelegt. In die Vorlage wurden 34.8 g Butylacrylat, 690 g VeoVa10 (Vinylester von α-verzweigten Monocarbonsäuren mit 10 C-Atomen) und 466 g Vinylacetat einemulgiert. Es wurde auf 50°C aufgeheizt und Ethylen mit einem Druck von 60 bar aufgedrückt. Nach Erreichen des Temperaturgleichgewichtes wurden eine Lösung von 11.7 g Ammoniumperoxodisulfat in 458 g Wasser mit 66 g/h und eine Lösung von 5.9 g Ascorbinsäure in 464 g Wasser mit 65 g/h zudosiert. Nach Auspolymerisieren der Vorlage wurde über einen Zeitraum von 5 Stunden ein Gemisch aus 4.7 kg Vinylacetat, 697 g VeoVa10 und 105 g Butylacrylat zudosiert. Eine Stunde vor Ablauf der Comonomerdosierung wurde eine wäßrige Lösung von 105 g N-Methylolacrylamid und 112 g Acrylamid zusammen mit der Comonomerdosierung zudosiert.
Nach Beendigung der Polymerisation resultierte eine wäßrige Dispersion mit einem Festgehalt von 52 Gew% und einer Copolymerzusammensetzung aus 11.1 Gew% Ethylen, 67.5 Gew% Vinylacetat, 18.3 Gew% VeoVa10, 1.8 Gew% Butylacrylat, 0.1 Gew% Vinylsulfonat, 0.5 Gew% Acrylamid und 0.7 Gew% N-Methylolacrylamid.

### Beispiel 4:

In einem Druckreaktor wurden 84.3 l Wasser, 1.28 kg einer 10 Gew%-igen wäßrigen Ameisensäurelösung, 108 kg einer 3.3 Gew%-igen wäßrigen Dispersion einer Hydroxyethylcellulose (Natrosol 250 GR), 20.5 kg einer 25 Gew%-igen Lösung eines Isotridecylalkohol-ethylenoxids (Genapol X360) und 580 g einer 25 Gew%-igen wäßrigen Vinylsulfonat-Lösung vorgelegt. In die Vorlage wurden 1.15 kg Butylacrylat, 22.7 kg VeoVa10 (Vinylester von α-verzweigten Monocarbonsäuren mit 10 C-Atomen) und 15.3 kg Vinylacetat einemulgiert. Es wurde auf 50°C aufgeheizt und Ethylen mit einem Druck von 60 bar aufgedrückt. Nach Erreichen des Temperaturgleichgewichtes wurden eine Lösung von 386 g Ammoniumperoxodisulfat in 15.1 kg Wasser und eine Lösung von 193.2 g Ascorbinsäure in 15.3 kg Wasser zudosiert. Nach Auspolymerisieren der Vorlage wurden über einen Zeitraum von 5 Stunden 155 kg Vinylacetat und ein Gemisch aus 22.9 kg VeoVa10 und 3.44 kg Butylacrylat zudosiert. Eine Stunde vor Ablauf der Comonomerdosierung wurde eine wäßrige Lösung von 3.22 kg N-Methylolacrylamid zusammen mit der Comonomerdosierung zudosiert.
Nach Beendigung der Polymerisation resultierte eine wäßrige Dispersion mit einem Festgehalt von 52 Gew% und einer Copolymerzusammensetzung aus 11.0 Gew% Ethylen, 68.0 Gew% Vinylacetat, 18.5 Gew% VeoValO, 1.7 Gew% Butylacrylat, 0.1 Gew% Vinylsulfonat, 0.7 Gew% N-Methylolacrylamid.

### Beispiel 5:

In einem Druckreaktor wurden 0.5 l Wasser, 1.01 g einer 30 Gew%-igen wäßrigen Lösung eines Sulfobernsteinsäurehalbesters (Aerosol A102), 9.39 g einer 20 Gew%-igen wäßrigen Lösung eines Ethylenoxid-Propylenoxid-Copolymerisats (Genapol PF40) und 2.43 g einer 25 Gew%-igen wäßrigen Vinylsulfonat-Lösung vorgelegt. In die Vorlage wurden 6.34 g Butylacrylat und 55.7 g Vinylacetat einemulgiert. Es wurde auf 50°C aufgeheizt und Ethylen mit einem Druck von 58 bar aufgedrückt. Nach Erreichen des Temperaturgleichgewichtes wurden eine Lösung von 6.99 g Ammoniumperoxodisulfat in 62.9 g Wasser und eine Lösung von 3.5 g Na-Formaldehydsulfoxylat (Brüggolit) in 66.7 g Wasser zudosiert. Nach Auspolymerisation der Vorlage wurden über einen Zeitraum von 4 Stunden 592 g Vinylacetat und eine Emulsion von 7.25 g Acrylsäure, 43.2 g Acrylamid und 79 g Genapol X360 in 43.8 g Wasser zudosiert. Eine Stunde vor Ablauf der Comonomerdosierung wurde eine wäßrige Lösung von 13.5 g N-Methylolacrylamid zusammen mit der Comonomerdosierung zudosiert.
Nach Beendigung der Polymerisation resultierte eine wäßrige Dispersion mit einem Festgehalt von 54 Gew% und einer Copolymerzusammensetzung aus 21.0 Gew% Ethylen, 76.5 Gew% Vinylacetat, 0.5 Gew% Butylacrylat, 0.1 Gew% Vinylsulfonat, 1.5 Gew% Acrylamid, 0.5 Gew% Acrylsäure und 0.8 Gew% N-Methylolacrylamid.

### Vergleichsbeispiel 3:

In einem Druckreaktor wurden 0.5 l Wasser, 1.02 g einer 30 Gew%-igen wäßrigen Lösung eines Sulfobernsteinsäurehalbesters (Aerosol A102), 9.45 g einer 20 Gew%-igen wäßrigen Lösung eines Ethylenoxid-Propylenoxid-Copolymerisats (Genapol PF40) und 2.45 g einer 25 Gew%-igen wäßrigen Vinylsulfonat-Lösung vorgelegt. In die Vorlage wurden 6.39 g Butylacrylat und 56.1 g Vinylacetat einemulgiert. Es wurde auf 50°C aufgeheizt und Ethylen mit einem Druck von 58 bar aufgedrückt. Nach Erreichen des Temperaturgleichgewichtes wurden eine Lösung von 7.04 g Ammoniumperoxodisulfat in 63.4 g Wasser und eine Lösung von 3.52 g Na-Formaldehydsulfoxylat (Brüggolit) in 67.1 g Wasser zudosiert. Nach Auspolymerisation der Vorlage wurden über einen Zeitraum von 4 Stunden 596 g Vinylacetat und eine Emulsion von 7.30 g Acrylsäure, 43.5 g Acrylamid und 79.5 g Genapol X360 in 44.1 g Wasser zudosiert.
Nach Beendigung der Polymerisation resultierte eine wäßrige Dispersion mit einem Festgehalt von 54 Gew% und einer Copolymerzusammensetzung aus 22.4 Gew% Ethylen, 74.5 Gew% Vinylacetat, 0.7 Gew% Butylacrylat, 0.1 Gew% Vinylsulfonat, 1.5 Gew% Acrylamid, 0.8 Gew% Acrylsäure.

### Anwendungstechnische Prüfung:

### Herstellung der Vliesstoffe:

Zur Herstellung der Vliesstoffe wurde ein Zellwollfaservlies mittels Vollbadimprägnierung mit 20 Gew% Polymerdispersion (Feststoff bezogen auf Faser) verfestigt. Der überschüssige Binder wurde zwischen zwei Walzen abgequetscht und das Vlies 3 Minuten bei 150°C in einem Trommeltrockner getrocknet.
Nach der Klimatisierung des etwa 30 g/m² schweren Vlieses bei 23°C und 50 % Luftfeuchtigkeit wurden der FormaldehydGehalt nach der Acetylaceton-Methode, die Festigkeit des Vlieses (Trockenfestigkeit und Naßfestigkeit) nach DIN 53857 und die Siegelbarkeit überprüft.

### Bestimmung des Formaldehyd-Gehalts nach der Acetylaceton-Methode:

2.5 g des Vlieses wurden zerkleinert und in einem Meßkolben mit 100 ml destilliertem Wasser 60 Minuten lang bei 40°C geschwenkt und anschließend filtriert. Das Filtrat wurde zur Formaldehyd-Bestimmung nach der Acetylaceton-Methode eingesetzt.

### Acetylaceton-Methode:

Das Acetylaceton-Reagenz bestand aus einer Mischung von 150 g Ammoniumacetat, 3 ml Eisessig und 2 ml Acetylaceton, welche mit destilliertem Wasser auf 1000 ml verdünnt wurde. 1 ml des verdünnten Überstands aus der Aufbereitung der Dispersion bzw. 5 ml des Filtrats aus der Aufbereitung des Vlieses wurden mit 5 ml Acetylaceton-Reagenz versetzt, 30 Minuten bei 50°C bzw. 40°C im Wasserbad geschüttelt, 30 Minuten bei Raumtemperatur stehengelassen und die Formaldehyd-Konzentration UV-spektrophotometrisch ermittelt.

### Bestimmung der Trocken- und Naßfestigkeit von Vliesen nach DIN 53857 (Höchstzugkraft und Dehnung):

Die Vliese wurden vor der Messung im Normalklima ausgelegt (Lufttemperatur 22°C, relative Luftfeuchte 55 %). Zur Bestimmung der Naßfestigkeit wurden die Vliese noch eine Minute in destilliertem Wasser gelagert. Aus den klimatisierten Vliesen wurden pro Messung je 10 Probestreifen (100 mm Länge, 15 mm Breite), fünf davon quer und fünf davon längs zur Fertigungsrichtung, entnommen. An einer Zugprüfmaschine wurden die Höchstzugkraft (HZK) und die Dehnung beim Vliesriß bestimmt und jeweils der Mittelwert aus den zehn Einzelprüfungen errechnet.

### Bestimmung der Siegelbarkeit der Vliese:

Die Siegelung erfolgte bei 180°C und einem Druck von 15 kp/cm². Die gesiegelte Fläche betrug 10 mm x 40 mm. Die Siegelung erfolgte dabei immer mit Oberseite gegen Oberseite.

Die Ergebnisse der anwendungstechnischen Prüfung sind in Tabelle 1 zusammengefaßt.

Die Ergebnisse aus Tabelle 1 zeigen den besonderen Vorteil der erfindungsgemäß hergestellten Polymerdispersionen. Mit sehr niederen Gehalten an N-Methylolacrylamid werden hohe Naßfestigkeiten und Siegelbarkeit erhalten, bei sehr geringer Formaldehyd-Emission aus dem gebundenen Vlies. Die Emissionen liegen in der Größenordnung der Grundbelastung des Vlieses. Wie niedrig die Formaldehyd-Belastung ist, zeigt auch der Vergleich mit Beispiel 5 oder Vergleichsbeispiel 3, in denen die Polymerisation in Gegenwart eines vielverwendeten Reduktionsmittels wie Brüggolit (Formaldehydsulfoxylat) durchgeführt wurde.

**TABELLE 1:**

| Ansatz | Höchstzugkraft [N] | | Siegelbarkeit | Formaldehydgehalt [ppm] |
|---|---|---|---|---|
| | trocken | naß | | |
| Beispiel 1 | 13.2 | 3.3 | ja | 7 |
| Beispiel 2 | 14.0 | 4.4 | ja | 8 |
| Beispiel 3 | 14.9 | 5.9 | ja | 7 |
| Beispiel 4 | 15.3 | 6.0 | ja | 8 |
| Beispiel 5 | 12.6 | 4.6 | ja | 34 |
| Vgl.beisp. 1 | 16.0 | 7.1 | nein | 6 |
| Vgl.beisp. 2 | 13.3 | 1.8 | ja | 7 |
| Vgl.beisp. 3 | 9.5 | 1.4 | ja | 45 |

## Patentansprüche

1. Verfahren zur Herstellung eines siegelbaren, selbstvernetzenden Bindemittels auf der Basis einer wäßrigen Dispersion von Polymerisaten enthaltend eines oder mehrere Monomere aus der Gruppe der Vinylester von unverzweigten oder verzweigten Alkylcarbonsäuren mit 1 bis 15 C-Atomen, welche 0.1 bis 5.0 Gew%, bezogen auf das Polymerisat, ethylenisch ungesättigte, vernetzend wirkende Comonomere aus der Gruppe der N-Alkylolamide und N-Alkoxyalkylamide ethylenisch ungesättigter Carbonsäuren und N-Methylolallylcarbamat enthalten, durch radikalische Polymerisation nach dem Emulsionspolymerisationsverfahren, dadurch gekennzeichnet, daß die Comonomeren des zu polymerisierenden Comonomergemisches, mit Ausnahme der vernetzend wirkenden Comonomerkomponente, teilweise vorgelegt und der Rest der vernetzerfreien Comonomermischung nach Initiierung der Polymerisation und Auspolymerisation der Vorlage zudosiert wird, und die Zugabe der ethylenisch ungesättigten, vernetzend wirkenden Comonomere erst ab dem Zeitpunkt erfolgt, zu dem mindestens 50 Gew% der nicht-vernetzenden Comonomere zum Polymerisationsansatz zudosiert worden sind.

2. Verfahren nach Anspruch 1, dadurch gekennzeichnet, daß die Emulsionspolymerisation bei einer Temperatur von 0 bis 80°C, in Gegenwart von 0.05 bis 3.0 Gew% wasserlöslichem Radikalbildner, 1 bis 6 Gew% Emulgator und 0 bis 15 Gew% Schutzkolloid so durchgeführt wird, daß die Zugabe der vernetzend wirkenden Comonomerkomponente ab dem Zeitpunkt erfolgt, zu dem 50 bis 99.5 Gew% der nicht-vernetzenden Comonomere zudosiert worden sind.

3. Verfahren nach Anspruch 1 bis 2, dadurch gekennzeichnet, daß als ethylenisch ungesättigte, vernetzend wirkende Comonomere 0.1 bis 2.0 Gew%, bezogen auf das Gesamtgewicht der Comonomerphase, N-Methylolacrylamid oder N-Methylolmethacrylamid zur Polymerisation eingesetzt werden.

4. Verfahren nach Anspruch 1 bis 3, dadurch gekennzeichnet, daß ein Comonomergemisch enthaltend 75 bis 95 Gew% Vinylester, 5 bis 25 Gew% Ethylen und 0.1 bis 2.0 Gew% N-Methylolacrylamid polymerisiert wird.

5. Verfahren nach Anspruch 1 bis 4, dadurch gekennzeichnet, daß ein Comonomergemisch enthaltend 50 bis 70 Gew% Vinylacetat, 10 bis 30 Gew% Vinylester einer α-verzweigten Carbonsäure, 5 bis 25 Gew% Ethylen, 0.1 bis 2.0 Gew% N-Methylolacrylamid und 0.1 bis 20 Gew% Acrylsäureester oder Diisopropylfumarat polymerisiert wird.

6. Verfahren nach Anspruch 1 bis 5, dadurch gekennzeichnet, daß das Comonomergemisch 0.05 bis 3.0 Gew%, bezogen auf das Gesamtgewicht des Comonomergemisches, ein oder mehrere Hilfsmonomere aus der Gruppe der ethylenisch ungesättigten Carbonsäuren, aus der Gruppe der ethylenisch ungesättigten Carbonsäureamide, aus der Gruppe der ethylenisch ungesättigten Sulfonsäuren bzw. deren Salze und aus der Gruppe der mehrfach ethylenisch ungesättigten Comonomeren enthält.

7. Bindemitteldispersion hergestellt nach einem Verfahren gemäß Anspruch 1 bis 6, dadurch gekennzeichnet, daß die Copolymerdispersion einen Festgehalt von 35 bis 65 Gew% hat und das Copolymerisat 75 bis 95 Gew% Vinylacetat, 5 bis 25 Gew% Ethylen und 0.1 bis 2.0 Gew% N-Methylolacrylamid enthält oder das Copolymerisat 50 bis 70 Gew% Vinylacetat, 10 bis 30 Gew% VeoVa9^{R} und/oder VeoVa10^{R}, 5 bis 25 Gew% Ethylen, 0.1 bis 2.0 Gew% N-Methylolacrylamid sowie 0.1 bis 20 Gew% Acrylsäureester oder Diisopropylfumarat enthält.

8. Verwendung der Bindemitteldispersionen nach Anspruch 1 bis 7 als Bindemittel für gewebte oder nicht gewebte flächige textile Gebilde aus natürlichen oder synthetischen Fasern.

## Claims

1. Process for the preparation of a sealable self-crosslinking binder based on an aqueous dispersion of polymers comprising one or more monomers from the group consisting of vinyl esters of unbranched or branched alkylcarboxylic acids having 1 to 15 C-atoms which comprise 0.1 to 5.0% by weight, based on the polymer, of ethylenically unsaturated comonomers having a crosslinking action from the group consisting of N-alkylolamides and N-alkoxyalkylamides of ethylenically unsaturated carboxylic acids and allyl N-methylolcarbamate, by free radical polymerization by the emulsion polymerization process, characterized in that a portion of the comonomers of the comonomer mixture to be polymerized, with the exception of the comonomer component having a crosslinking action, is initially introduced into the reaction vessel and the remainder of the crosslinker-free comonomer mixture is metered in after initiation of the polymerization and complete polymerization of the initial mixture, and the addition of the ethylenically unsaturated comonomers having a crosslinking action takes place only after the point in time at which at least 50% by weight of the non-crosslinking comonomers has been metered in to the polymerization batch.

2. Process according to Claim 1, characterized in that the emulsion polymerization is carried out at a temperature of 0 to 80°C in the presence of 0.05 to 3.0% by weight of a water-soluble agent which forms free radicals, 1 to 6% by weight of an emulsifier and 0 to 15% by weight of a protective colloid such that the addition of the comonomer component having a crosslinking action is added after the point in time at which 50 to 99.5% by weight of the non-crosslinking comonomers have been metered in.

3. Process according to Claims 1 to 2, characterized in that 0.1 to 2.0% by weight, based on the total weight of the comonomer phase, of N-methylolacrylamide or N-methylolmethacrylamide is employed as the ethylenically unsaturated comonomer having a crosslinking action for the polymerization.

4. Process according to Claims 1 to 3, characterized in that a comonomer mixture comprising 75 to 95% by weight of vinyl ester, 5 to 25% by weight of ethylene and 0.1 to 2.0% by weight of N-methylolacrylamide is polymerized.

5. Process according to Claims 1 to 4, characterized in that a comonomer mixture comprising 50 to 70% by weight of vinyl acetate, 10 to 30% by weight of vinyl ester of an α-branched carboxylic acid, 5 to 25% by weight of ethylene, 0.1 to 2.0% by weight of N-methylolacrylamide and 0.1 to 20% by weight of an acrylic acid ester or diisopropyl fumarate is polymerized.

6. Process according to Claims 1 to 5, characterized in that the comonomer mixture comprises 0.05 to 3.0% by weight, based on the total weight of the comonomer mixture, of one or more auxiliary monomers from the group consisting of ethylenically unsaturated carboxylic acids, from the group consisting of ethylenically unsaturated carboxylic acid amides, from the group consisting of ethylenically unsaturated sulphonic acids and salts thereof and from the group consisting of poly-ethylenically unsaturated comonomers.

7. Binder dispersion prepared by a process according to Claims 1 to 6, characterized in that the copolymer dispersion has a solids content of 35 to 65% by weight and the copolymer comprises 75 to 95% by weight of vinyl acetate, 5 to 25% by weight of ethylene and 0.1 to 2.0% by weight of N-methylolacrylamide, or the copolymer comprises 50 to 70% by weight of vinyl acetate, 10 to 30% by weight of VeoVa9^{R} and/or VeoVa10^{R}, 5 to 25% by weight of ethylene, 0.1 to 2.0% by weight of N-methylolacrylamide and 0.1 to 20% by weight of an acrylic acid ester or diisopropylfumarate.

8. Use of the binder dispersions according to Claims 1 to 7 as binders for woven or nonwoven flat textile structures of naturally occurring or synthetic fibres.

## Revendications

1. Procédé de préparation d'un liant autoréticulant scellable à base d'une dispersion aqueuse de polymères contenant un ou plusieurs monomères du groupe des esters vinyliques d'acides alcoylcarboxyliques linéaires ou branchés avec 1 à 15 atomes de carbone, lesquels contiennent de 0,1 à 5,0% en poids, sur base du polymère, de comonomères éthyléniquement insaturés, agissant comme réticulants, parmi le groupe des N-alcoylolamides et des N-alcoxyalcoylamides d'acides carboxyliques éthyléniquement insaturés et du N-méthylolallylcarbamate, par polymérisation radicalaire d'après un procédé de polymérisation par émulsion, caractérisé en ce qu'une partie des comonomères du mélange de comonomères à polymériser, à l'exception des composants comonomères agissant comme réticulants, est mise à réagir et le reste du mélange de comonomères exempt de réticulant est ajouté après initiation de la polymérisation et polymérisation de la partie préalable, et l'addition des comonomères éthyléniquement insaturés, agissant comme réticulants, se fait à partir du moment où au moins 50% en poids des comonomères non réticulants ont été ajoutés à l'opération de polymérisation.

2. Procédé suivant la revendication 1, caractérisé en ce que la polymérisation par émulsion est réalisée à une température de 0 à 80°C, en présence de 0,05 à 3,0% en poids d'un agent formant des radicaux, soluble dans l'eau, 1 à 6% en poids d'un émulsionnant et 0 à 15% en poids d'un colloïde protecteur, de sorte que l'addition des composants comonomères agissant comme réticulants se fait à partir du moment où de 50 à 99,5% en poids des comonomères non réticulants ont été ajoutés.

3. Procédé suivant les revendications 1 à 2, caractérisé en ce que l'on ajoute à la polymérisation, comme comonomères éthyléniquement insaturés, agissant comme réticulants, de 0,1 à 2,0% en poids, sur base du poids total de la phase comonomère, de N-méthylolacrylamide ou de N-méthylolméthacrylamide.

4. Procédé suivant les revendications 1 à 3, caractérisé en ce que l'on polymérise un mélange comonomère contenant 75 à 95% en poids d'ester vinylique, 5 à 25% en poids d'éthylène et 0,1 à 2,0% en poids de N-méthylolacrylamide.

5. Procédé suivant les revendications 1 à 4, caractérisé en ce que l'on polymérise un mélange comonomère contenant 50 à 70% en poids d'acétate de vinyle, 10 à 30% en poids d'ester vinylique d'un acide carboxylique α-ramifié, 5 à 25% en poids d'éthylène et 0,1 à 2,0% en poids de N-méthylolacrylamide et 0,1 à 20% en poids d'ester de l'acide acrylique ou de fumarate de diisopropyle.

6. Procédé suivant les revendications 1 à 5, caractérisé en ce que le mélange comonomère contient 0,05 à 3,0% en poids, sur base du poids total du mélange comonomère, d'un ou plusieurs monomères auxiliaires parmi le groupe des acides carboxyliques éthyléniquement insaturés, parmi le groupe des amides d'acides carboxyliques éthyléniquement insaturés, parmi le groupe des acides sulfoniques éthyléniquement insaturés et respectivement, leurs sels et parmi le groupe des comonomères à plusieurs insaturations éthyléniques.

7. Dispersion d'agent liant préparée d'après un procédé suivant les revendications 1 à 6, caractérisée en ce que la dispersion copolymère a une teneur en matière solide de 35 à 65% en poids et que le copolymère contient 75 à 95% en poids d'acétate de vinyle, 5 à 25% en poids d'éthylène et 0,1 à 2,0% en poids de N-méthylolacrylamide ou que le copolymère contient 50 à 70% en poids d'acétate de vinyle, 10 à 30% en poids de VeoVa9^{®} et/ou de Veova10^{®}, 5 à 25% en poids d'éthylène et 0,1 à 2,0% en poids de N-méthylolacrylamide, ainsi que 0,1 à 20% en poids d'ester d'acide acrylique ou de fumarate de diisopropyle.

8. Utilisation des dispersions d'agent liant suivant les revendications 1 à 7, comme liant pour des structures textiles planes tissées ou non tissées, constituées de fibres naturelles ou synthétiques.
